# EUROPEAN PATENT SPECIFICATION

(11) **EP 3 826 599 B1**
(45) Date of publication and mention of the grant of the patent: **06.09.2023**
(21) Application number: 19769893.9
(22) Date of filing: 24.07.2019
(51) Int. Cl.: G16H 40/20, A61H 31/00, A61M 1/36, A61N 1/39

(54) **MEDICAL EMERGENCY EQUIPMENT**
MEDIZINISCHE NOTAUSRÜSTUNG
ÉQUIPEMENT MÉDICAL D'URGENCE

(30) Priority: 26.07.2018 IT 201800007542
(43) Date of publication of application: 02.06.2021
(73) Proprietor: Eurosets S.r.l., 41036 Medolla (MO) (IT)
(72) Inventor: FONTANILI, Paolo, 41036 Medolla (MO) (IT); GHELLI, Nicola, 41036 Medolla (MO) (IT); PETRALIA, Antonio, 41036 Medolla (MO) (IT)
(74) Representative: Zoli, Filippo
(86) International application number: PCT/IB2019/056324
(87) International publication number: WO 2020/021469

(56) References cited:
- US-A- 5 494 051
- US-A1- 2001 016 696
- US-A1- 2011 172 550
- US-A1- 2015 231 027
- US-A1- 2016 321 480

## Description

### Technical Field

The present invention relates to medical emergency equipment.

### Background Art

Various types of first aid medical equipment are known to be used, e.g., in the event of accidents, traumatic events or sudden pathological events.

A known type of equipment is related to the treatment of patients with a cardiac arrest.

This type of equipment has a load-bearing frame which defines a supporting surface of the patient's back, and on which mechanical means for cardiac massage are applied. These mechanical means are adapted to exert pressure on the patient's chest, with a frequency that varies according to the patient's condition.

The use of this type of equipment is however limited in time, i.e. it is useful only in the moments immediately following cardiac arrest, as it does not allow the patient to be kept alive.

This implies that the patient must necessarily be transported urgently to a hospital in order to allow the medical staff to perform the necessary interventions to keep the patient alive and to restore a proper clinical picture. US 2015/231027 A1 discloses a medical emergency equipment having a load-bearing frame defining a supporting surface for a patient's back and one of a defibrillator and means for cardiac massage associated with the frame.

### Description of the Invention

The main aim of the present invention is to devise medical emergency equipment that allows offering a therapeutic support to the patient over a long period of time compared to the equipment of known type.

In particular, the equipment to which the present invention relates aims to keep the patient alive at the same time and/or after resuscitation, in order to allow medical staff to intervene promptly even during the transport of the patient to a hospital.

Within this aim, one object of the present invention is to devise a piece of equipment that is easily transportable and allows assisting the action of the means for cardiac massage.

Another object of the present invention is to devise medical emergency equipment that allows overcoming the aforementioned drawbacks of the prior art in a simple, rational, easy, effective to use as well as cost-effective solution. The aforementioned objects are achieved by the present medical emergency equipment according to claim 1.

### Brief Description of the Drawings

Other characteristics and advantages of the present invention will become more evident from the description of a preferred, but not exclusive, embodiment of medical emergency equipment, illustrated by way of non-limitative example in the accompanying tables of drawings in which:

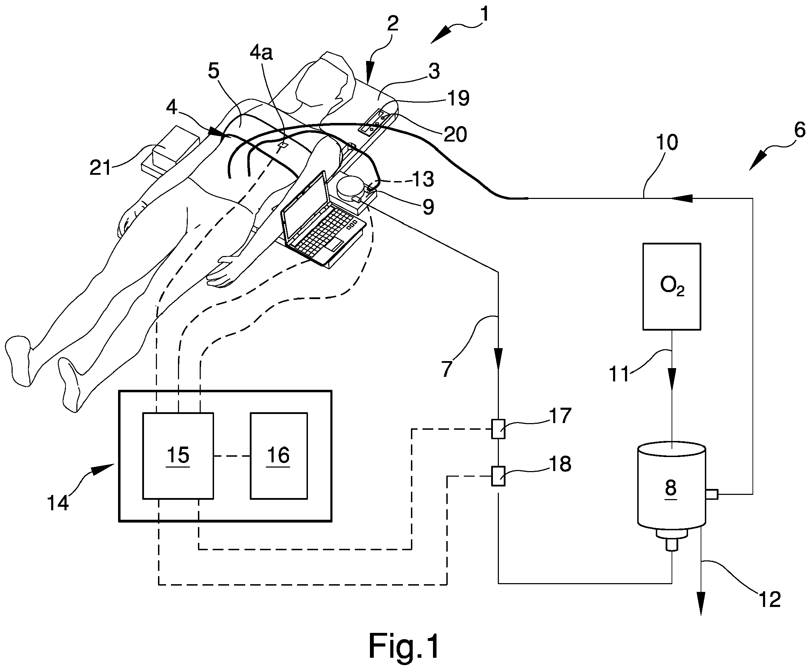

Figure 1 is an axonometric view of the equipment according to the invention.

### Embodiments of the Invention

With particular reference to these figures, reference numeral 1 globally indicates a type of medical emergency equipment.

The equipment 1 comprises a load-bearing frame 2, which defines a supporting surface 3 for a patient's back.

The frame 2 has an elongated and substantially slab-shaped conformation, so that it can be easily transported.

The frame 2 is associated with at least one of a defibrillator 21 and means for cardiac massage 4.

Therefore, embodiments are possible which provide for the presence of only one of the defibrillator 21 and the means for cardiac massage 4 and others, such as the one shown in the figures, which provide for the simultaneous presence of both the defibrillator 21 and the means for cardiac massage 4.

Preferably, the means for cardiac massage 4 comprise at least one pusher element 5 which is adapted to operate on the patient's chest in order to reactivate the heartbeat. More particularly, the pusher element 5 can be of the type of a band that wraps around the patient's chest, and that is moved mechanically so as to exert pressure on the patient him/herself, or a single element movable alternately in translation and adapted to operate on a circumscribed area of the patient. Alternative embodiments of the means for cardiac massage known to the technician of the sector cannot however be ruled out.

Appropriately, sensor means 4a are provided which are adapted to detect the patient's heartbeat. In the embodiment shown in the figures, the sensor means 4a are built in the means for cardiac massage 4; alternative embodiments wherein the sensor means 4a are separate from the means for cardiac massage 4 cannot however be ruled out.

According to the invention, the equipment 1 also comprises a circuit 6 for the extracorporeal circulation of the patient's blood, which is associated with the frame 2 and operatively connected to the defibrillator 21 and/or to the means for cardiac massage 4.

In particular, the circuit 6 comprises at least one venous line 7 connectable to the patient to take the blood to be oxygenated, at least one oxygenator 8 which is adapted to supply oxygen to the blood and/or to remove carbon dioxide from the same, where along the venous line 7 are located blood pumping means 9, and at least one arterial line 10 connected at outlet to the oxygenator 8 and adapted to bring the oxygenated blood back to the patient.

The oxygenator 8 comprises at least one inlet port for the blood to be oxygenated connected to the venous line 7 and at least one outlet port for the oxygenated blood connected to the arterial line 10, at least one inlet channel 11 and at least one outlet channel 12 of a work gas, such as air or oxygen, intended to supply oxygen to the blood and/or to remove carbon dioxide from the same. The pumping means 9 comprise at least one driving motor, not visible in detail in the figures, and the frame 2 has at least one seat 13 for housing the driving motor itself.

Advantageously, the equipment 1 comprises at least one electronic control unit 14 programmed to control the circuit 6 for extracorporeal circulation and at least one of a defibrillator 21 and means for cardiac massage 4. In particular, the electronic control unit 14 is adapted to manage the transitional phase occurring between the use of the defibrillator 21 and/or the means for cardiac massage 4 and the autonomous operation of the circuit 6.

More in detail, the electronic control unit 14 is provided with a processing unit 15, of the type of a microprocessor, which is adapted to receive the signal from the sensor means 4a that detect the heart rate of the patient.

Preferably, the electronic control unit 14, and in particular the processing unit 15, is operatively connected to the pumping means 9, and in particular to its driving motor, in such a way as to regulate the flow rate of the blood flowing through the circuit 6.

In the embodiment that provides for the presence of means for cardiac massage 4, the processing unit 15 is adapted to control the movement of the pusher element 5 in order to increase or decrease the rate at which it interacts with the patient. Appropriately, the electronic control unit 14 is also provided with a programmable memory 16 which is operatively connected to the processing unit 15 and inside which reference pressure values and/or flow values can be set. Advantageously, along the circuit 6 are arranged at least one pressure sensor 17 and/or at least one flow rate sensor 18 which are adapted to detect the pressure and flow rate respectively of the blood flowing through the circuit itself and operatively connected to the processing unit 15. Preferably, along the circuit 6 are arranged both the pressure sensor 17 and the flow rate sensor 18.

The processing unit 15 is programmed to compare the pressure and/or flow rate values detected by the sensors 17 and 18 with the reference pressure and/or flow rate values set in the programmable memory 16, respectively.

The processing unit 15 is adapted to calculate the difference between the reference pressure and/or flow rate values and the pressure and/or flow rate values detected by the sensors 17 and 18 and to operate on said pusher element 5 and on said pumping means 9 depending on the calculated difference. More in detail, the processing unit 15 is adapted to reduce or increase the frequency of movement of the pusher element 5 as the calculated difference decreases or increases, respectively. In fact, if the difference between the reference pressure and/or flow rate values and the values detected by the sensors 17 and 18 increases, it means that the extracorporeal circulation is not yet able to independently support the patient's vital functions, so it is necessary to increase the action of the means for cardiac massage 4. Similarly, when the difference calculated by the processing unit 15 decreases, it means that the extracorporeal circulation is increasing its efficiency, so the action of the means for cardiac massage 4 can be interrupted.

The means for cardiac massage 4 and the circuit 6 can operate simultaneously as described above only if they are synchronized with the heartbeat.

When the pressure and/or flow rate values detected by the sensors 17 and 18 are equal to the respective reference values, the processing unit 15 deactivates the action of the pusher element 5 since the circuit 6 is able to support the patient's vital functions independently.

If the pressure and/or flow rate values detected by the sensors 17 and 18 exceed the reference pressure and/or flow rate values, it means that the demand of the circuit 6 is greater than the patient's needs, so the processing unit 15 is adapted to intervene on the pumping means 9 in order to reduce the flow rate of the blood flowing through the circuit 6.

Preferably, the electronic control unit 14 is operatively connected to a display 19 and the processing unit 15 is adapted to receive the data detected by the sensors 4a, 17 and 18 to show them at least partly on the display 19 in a graphic and/or numerical form.

In the embodiment that provides for the presence of the defibrillator 21 only, the processing unit 15 is adapted to keep deactivated the pumping means 9 during the operation of the defibrillator 21. When the processing unit 15 detects the presence of the heartbeat by means of the signal received from the sensor means 4a, the unit disables the operation of the defibrillator 21 and activates the pumping means 9 so as to allow extracorporeal circulation flowing through the circuit 6.

In the embodiment which provides for the simultaneous presence of the defibrillator 21 and of the means for cardiac massage 4, the processing unit 15 is adapted to keep deactivated both the means for cardiac massage themselves and the pumping means 9 during the operation of the defibrillator 21. When the processing unit 15 detects the presence of the heartbeat by means of the signal received from the sensor means 4a, the unit disables the operation of the defibrillator 21 and activates at least one of the means for cardiac massage 4 and the pumping means 9.

The equipment 1 also comprises manual activation means 20 of at least one of the defibrillator 21 and the means for cardiac massage 4, so that the medical staff can activate the operation thereof once the patient has been placed in the correct position, and the pumping means 9, so that the medical operator can activate the extracorporeal circulation after the circuit 6 has been properly assembled and the venous and arterial lines 7 and 10 have been connected to the patient.

The operation of the present invention is as follows.

When there is an urgent need to assist a patient with a cardiac arrest, the medical staff shall first of all place the patient safely on the supporting surface 3 so that his or her chest is placed below the pusher element 5.

Then, depending on the equipment 1, the medical operator activates one of the defibrillator 21 and the means for cardiac massage 4 by means of manual activation means 20, so as to reactivate the patient's cardiac function as soon as possible.

If the equipment 1 is provided with both, the medical operator can decide, based on the patient's condition, whether to activate only one of them or whether to activate the defibrillator 21 first and then the means for cardiac massage 4.

While the defibrillator 21 or the means for cardiac massage 4 are in operation, the medical operator, after the circuit 6 has been assembled, connects the venous line 7 and the arterial line 10 to the patient, after which he/she intervenes on the activation means 20 to activate the driving motor, which has been suitably positioned inside the seat 13, and consequently the extracorporeal circulation.

As mentioned above, in the event of the defibrillator 21 being active, the electronic control unit 14 is adapted to prevent the activation of the pumping means 9 until the defibrillator itself is active.

If, on the other hand, the means for cardiac massage 4 are active, the electronic control unit 14 is programmed, if necessary, to manage the transient, i.e. the progressive intervention of the extracorporeal circulation through the circuit 6. The possible simultaneous operation of the means for cardiac massage 4 and the circuit 6 is synchronized with the patient's heartbeat.

It has in practice been found that the described invention achieves the intended objects and in particular the fact is underlined that the equipment to which the present invention relates allows intervening promptly on a patient suffering from cardiac arrest both by restoring the cardiac activity thereof and at the same time allowing medical staff to carry out surgical interventions or pharmacological therapies. By means of the extracorporeal blood circulation it is in fact possible to stabilize the patient's condition, thus allowing medical staff to implement the most suitable therapies in a very short time and without having to wait to reach a fully equipped hospital.

In addition, the equipment according to the invention allows modulating the activity of the means for cardiac massage according to the parameters of extracorporeal circulation, so as to optimize the operation of both and ensure good therapeutic support to the patient while maintaining synchronization with the patient's heartbeat.

## Claims

1. Medical emergency equipment (1), comprising:
- a load-bearing frame (2) defining at least one supporting surface (3) for a patient's back;
- at least one of a defibrillator (21) and means for cardiac massage (4) associated with said frame (2); the equipment is **characterised by**:
- a circuit (6) for extracorporeal circulation of the patient's blood associated with said frame (2)
and operatively connected to said at least one of a defibrillator (21) and means for cardiac massage (4)
- one electronic control unit (14) programmed to control said at least one of a defibrillator (21) and means for cardiac massage (4) and to control said circuit (6) for the extracorporeal circulation, said electronic control unit (14) being adapted to manage the transitional phase occurring between the use of said defibrillator (21) and/or said means for cardiac massage (4) and the autonomous operation of said circuit (6).

2. Equipment (1) according to claim 1, **characterized by** the fact that said circuit (6) for the extracorporeal circulation comprises at least one oxygenator (8) which is adapted to supply oxygen to the blood and/or to remove carbon dioxide from it, at least one venous line (7) which is adapted to take blood to be oxygenated from the patient and connected at inlet to said oxygenator (8), along said venous line (7) being located blood pumping means (9), and at least one arterial line (10) connected at outlet to said oxygenator (8) and adapted to bring the oxygenated blood back to the patient,
and **characterized by** the fact that said electronic control unit (14) is operatively connected to said at least one of a defibrillator (21) and means for cardiac massage (4) and to said pumping means (9).

3. Equipment (1) according to claim 1 or 2, **characterized by** the fact that it comprises said means for cardiac massage (4) and by the fact that said electronic control unit (14) is operatively connected to said circuit (6) for the extracorporeal circulation and to said means for cardiac massage (4).

4. Equipment (1) according to one or more of the preceding claims, **characterized by** the fact that said electronic control unit (14) comprises at least one programmable memory (16) with at least one reference pressure value and/or one flow rate value and at least one data processing unit (15) operatively connected to said programmable memory (16), and by the fact that it comprises at least one pressure sensor (17) and/or at least one flow rate sensor (18) located along said extracorporeal circulation circuit (6) operatively connected to said data processing unit (15), the latter being programmed to compare the pressure and/or flow rate values detected by said sensors (17, 18) with said reference pressure and/or flow rate values, respectively.

5. Equipment (1) according to one or more of the preceding claims, **characterized by** the fact that said means for cardiac massage (4) comprise at least one pusher element (5) which is adapted to operate on the patient's chest and by the fact that said processing unit (15) is programmed to calculate the difference between said reference pressure and/or flow rate values and said pressure and/or flow rate values detected by said sensors (17, 18) and to reduce or increase the frequency of movement of said pusher element (5) as said calculated difference decreases and increases, respectively.

6. Equipment (1) according to claim 4 or 5, **characterized by** the fact that said electronic control unit (14) is operatively connected to a display (19) and by the fact that said processing unit (15) is adapted to receive the data detected by said sensors (17, 18) to show them at least partly on said display (19) in a graphic and/or numerical form.

7. Equipment (1) according to one or more of claims from 3 to 6, **characterized by** the fact that it comprises said defibrillator (21) and by the fact that said electronic control unit (14) is operatively connected to said circuit (6) for the extracorporeal circulation, to said means for cardiac massage (4) and to said defibrillator (21).

8. Equipment (1) according to claim 7, **characterized by** the fact that said electronic control unit (14) is adapted to deactivate at least said pumping means (9) during the operation of said defibrillator (21).

9. Equipment (1) according to one or more of the preceding claims, **characterized by** the fact that it comprises manual activation means (20) for activating at least one of said pumping means (9), said defibrillator (21) and said means for cardiac massage (4).

10. Equipment (1) according to one or more of the preceding claims, **characterized by** the fact that said pumping means (9) comprise at least one driving motor and by the fact that said frame (2) comprises at least one seat (13) for housing said driving motor.

## Patentansprüche

1. Medizinische Notfallausrüstung (1), aufweisend:
einen tragenden Rahmen (2), der mindestens eine Auflagefläche (3) für den Rücken eines Patienten definiert;
mindestens einen Defibrillator (21) und Mittel zur Herzmassage (4), die dem Rahmen (2) zugeordnet sind;
wobei die Ausrüstung **gekennzeichnet ist durch**:
einen Kreislauf (6) für die extrakorporale Zirkulation des Blutes des Patienten, der dem Gestell (2) zugeordnet ist und mit dem Defibrillator (21) und/oder den Mitteln für die Herzmassage (4) in Wirkverbindung steht,
eine elektronische Steuereinheit (14), die programmiert ist, um den Defibrillator (21) und/oder die Mittel zur Herzmassage (4) zu steuern und den Kreislauf (6) für die extrakorporale Zirkulation zu steuern, wobei die elektronische Steuereinheit (14) ausgebildet ist, um die Übergangsphase zu steuern, die zwischen der Verwendung des Defibrillators (21) und/oder der Mittel zur Herzmassage (4) und dem autonomen Betrieb des Kreislaufs (6) auftritt.

2. Ausrüstung (1) nach Anspruch 1, **dadurch gekennzeichnet, dass** der Kreislauf (6) für den extrakorporalen Kreislauf mindestens einen Oxygenator (8) aufweist, der ausgebildet ist, um dem Blut Sauerstoff zuzuführen und/oder ihm Kohlendioxid zu entziehen, mindestens eine venöse Leitung (7), die ausgebildet ist, um dem Patienten das mit Sauerstoff zu versorgende Blut zu entnehmen, und die am Eingang mit dem Oxygenator (8) verbunden ist, wobei entlang der venösen Leitung (7) Blutpumpmittel (9) angeordnet sind, und mindestens eine arterielle Leitung (10), die am Ausgang mit dem Oxygenator (8) verbunden ist und ausgebildet ist, um das mit Sauerstoff versorgte Blut zum Patienten zurückzubringen, und **dadurch gekennzeichnet, dass** die elektronische Steuereinheit (14) mit dem Defibrillator (21) und/oder den Mitteln zur Herzmassage (4) und mit den Pumpmitteln (9) in Wirkverbindung steht.

3. Ausrüstung (1) nach Anspruch 1 oder 2, **dadurch gekennzeichnet, dass** sie die Mittel zur Herzmassage (4) aufweist und dass die elektronische Steuereinheit (14) mit dem Kreislauf (6) für den extrakorporalen Kreislauf und den Mitteln zur Herzmassage (4) in Wirkverbindung steht.

4. Ausrüstung (1) nach einem oder mehreren der vorhergehenden Ansprüche, **dadurch gekennzeichnet, dass** die elektronische Steuereinheit (14) mindestens einen programmierbaren Speicher (16) mit mindestens einem Referenzdruckwert und/oder einem Durchflusswert und mindestens eine Datenverarbeitungseinheit (15) aufweist, die mit dem programmierbaren Speicher (16) in Wirkverbindung steht, und dadurch, dass sie mindestens einen Drucksensor (17) und/oder mindestens einen Durchflusssensor (18) aufweist, die entlang des extrakorporalen Kreislaufs (6) angeordnet und mit der Datenverarbeitungseinheit (15) verbunden sind, wobei letztere programmiert ist, um die von den Sensoren (17, 18) erfassten Druck- und/oder Durchflusswerte mit den jeweiligen Referenzdruck- und/oder Durchflusswerten zu vergleichen.

5. Ausrüstung (1) nach einem oder mehreren der vorhergehenden Ansprüche, **dadurch gekennzeichnet, dass** die Mittel zur Herzmassage (4) mindestens ein Drückelement (5) aufweisen, das ausgebildet ist, um auf die Brust des Patienten einzuwirken, und dass die Datenverarbeitungseinheit (15) programmiert ist, um die Differenz zwischen den Werten des Referenzdrucks und/oder der Durchflussmenge und den von den Sensoren (17, 18) erfassten Werten des Drucks und/oder der Durchflussmenge zu berechnen und die Frequenz der Bewegung des Drückelements (5) zu verringern oder zu erhöhen, wenn die berechnete Differenz abnimmt bzw. zunimmt.

6. Ausrüstung (1) nach Anspruch 4 oder 5, **dadurch gekennzeichnet, dass** die elektronische Steuereinheit (14) operativ mit einer Anzeige (19) verbunden ist und dass die Datenverarbeitungseinheit (15) ausgebildet ist, die von den Sensoren (17, 18) erfassten Daten zu empfangen, um sie zumindest teilweise auf der Anzeige (19) in einer grafischen und/oder numerischen Form anzuzeigen.

7. Ausrüstung (1) nach einem oder mehreren der Ansprüche 3 bis 6, **dadurch gekennzeichnet, dass** sie den Defibrillator (21) aufweist und dass die elektronische Steuereinheit (14) mit dem Kreislauf (6) für den extrakorporalen Kreislauf, mit den Mitteln für die Herzmassage (4) und mit dem Defibrillator (21) in Wirkverbindung steht.

8. Ausrüstung (1) nach Anspruch 7, **dadurch gekennzeichnet, dass** die elektronische Steuereinheit (14) ausgebildet ist, um zumindest die Pumpmittel (9) während des Betriebs des Defibrillators (21) zu deaktivieren.

9. Ausrüstung (1) nach einem oder mehreren der vorhergehenden Ansprüche, **dadurch gekennzeichnet, dass** sie manuelle Aktivierungsmittel (20) aufweist, um die Pumpmittel (9) und/oder den Defibrillator (21) und/oder die Mittel zur Herzmassage (4) zu aktivieren.

10. Ausrüstung (1) nach einem oder mehreren der vorhergehenden Ansprüche, **dadurch gekennzeichnet, dass** die Pumpmittel (9) mindestens einen Antriebsmotor aufweisen und dass der Rahmen (2) mindestens einen Sitz (13) zur Aufnahme des Antriebsmotors aufweist.

## Revendications

1. - Équipement médical d'urgence (1), comprenant :
- un châssis porteur de charge (2) définissant au moins une surface de support (3) pour le dos d'un patient ;
- au moins un défibrillateur (21) et des moyens de massage cardiaque (4) associés audit châssis (2) ;
l'équipement étant **caractérisé par** :
- un circuit (6) de circulation extracorporelle de sang du patient, associé audit châssis (2) et relié de manière fonctionnelle à au moins un d'un défibrillateur (21) et de moyens de massage cardiaque (4),
- une unité de commande électronique (14) programmée pour commander ledit au moins un parmi le défibrillateur (21) et les moyens de massage cardiaque (4) et pour commander ledit circuit (6) de circulation extracorporelle, ladite unité de commande électronique (14) étant agencée pour gérer la phase transitoire se produisant entre l'utilisation dudit défibrillateur (21) et/ou desdits moyens de massage cardiaque (4) et le fonctionnement autonome dudit circuit (6).

2. - Equipement (1) selon la revendication 1, **caractérisé par le fait que** ledit circuit (6) de circulation extracorporelle comprend au moins un oxygénateur (8) qui est agencé pour fournir de l'oxygène au sang et/ou retirer le dioxyde de carbone de celui-ci, au moins une ligne veineuse (7) qui est agencée pour prélever du sang à oxygéner à partir du patient et reliée à l'entrée dudit oxygénateur (8), le long de ladite ligne veineuse (7) étant situés des moyens de pompage de sang (9), et au moins une ligne artérielle (10) reliée à la sortie dudit oxygénateur (8) et agencée pour ramener le sang oxygéné au patient,
et **caractérisé par le fait que** ladite unité de commande électronique (14) est reliée de manière fonctionnelle audit au moins un parmi le défibrillateur (21) et les moyens de massage cardiaque (4), et auxdits moyens de pompage (9).

3. - Equipement (1) selon la revendication 1 ou 2, **caractérisé par le fait qu'**il comprend lesdits moyens de massage cardiaque (4) et **par le fait que** ladite unité de commande électronique (14) est reliée de manière fonctionnelle audit circuit (6) de circulation extracorporelle et auxdits moyens de massage cardiaque (4).

4. - Equipement (1) selon une ou plusieurs des revendications précédentes, **caractérisé par le fait que** ladite unité de commande électronique (14) comprend au moins une mémoire programmable (16) ayant au moins une valeur de pression de référence et/ou une valeur de débit et au moins une unité de traitement de données (15) reliée de manière fonctionnelle à ladite mémoire programmable (16), et **par le fait qu'**il comprend au moins un capteur de pression (17) et/ou au moins un capteur de débit (18) situé le long dudit circuit de circulation extracorporelle (6) relié de manière fonctionnelle à ladite unité de traitement de données (15), cette dernière étant programmée pour comparer les valeurs de pression et/ou de débit détectées par lesdits capteurs (17, 18) auxdites valeurs de pression de référence et/ou de débit, respectivement.

5. - Equipement (1) selon une ou plusieurs des revendications précédentes, **caractérisé par le fait que** lesdits moyens de massage cardiaque (4) comprennent au moins un élément pousseur (5) qui est agencé pour agir sur la poitrine du patient, et **par le fait que** ladite unité de traitement (15) est programmée pour calculer la différence entre lesdites valeurs de pression de référence et/ou de débit et lesdites valeurs de pression et/ou de débit détectées par lesdits capteurs (17, 18) et pour réduire ou augmenter la fréquence de mouvement dudit élément pousseur (5) à mesure que ladite différence calculée diminue et augmente, respectivement.

6. - Equipement (1) selon la revendication 4 ou 5, **caractérisé par le fait que** ladite unité de commande électronique (14) est reliée de manière fonctionnelle à un dispositif d'affichage (19) et **par le fait que** ladite unité de traitement (15) est agencée pour recevoir les données détectées par lesdits capteurs (17, 18) pour les afficher au moins en partie sur ledit dispositif d'affichage (19) sous une forme graphique et/ou numérique.

7. - Equipement (1) selon une ou plusieurs des revendications 3 à 6, **caractérisé par le fait qu'**il comprend ledit défibrillateur (21) et **par le fait que** ladite unité de commande électronique (14) est reliée de manière fonctionnelle audit circuit (6) de circulation extracorporelle, auxdits moyens de massage cardiaque (4) et audit défibrillateur (21).

8. - Equipement (1) selon la revendication 7, **caractérisé par le fait que** ladite unité de commande électronique (14) est agencée pour désactiver au moins lesdits moyens de pompage (9) pendant le fonctionnement dudit défibrillateur (21).

9. - Equipement (1) selon une ou plusieurs des revendications précédentes, **caractérisé par le fait qu'**il comprend des moyens d'activation manuels (20) pour activer au moins un desdits moyens de pompage (9), dudit défibrillateur (21) et desdits moyens de massage cardiaque (4) .

10. - Equipement (1) selon une ou plusieurs des revendications précédentes, **caractérisé par le fait que** lesdits moyens de pompage (9) comprennent au moins un moteur d'entraînement et **par le fait que** ledit châssis (2) comprend au moins un siège (13) pour recevoir ledit moteur d'entraînement.
